# EUROPÄISCHE PATENTSCHRIFT

(11) **EP 1 127 546 B1**
(45) Veröffentlichungstag und Bekanntmachung des Hinweises auf die Patenterteilung: **08.03.2006**
(21) Anmeldenummer: 01200652.4
(22) Anmeldetag: 22.02.2001
(51) Int. Cl.: A61B 6/03

(54) **Computertomograph zur Ermittlung des Impulsübertrags-Spektrums in einem Untersuchungsbereich**
CT apparatus for detecting the spectrum of pulse transmission in an inspection field
Tomographe assisté par ordinateur pour détecter le spectre de transmission d'impulsions dans une zone d'inspection

(30) Priorität: 28.02.2000 DE 10009285
(43) Veröffentlichungstag der Anmeldung: 29.08.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Harding, Geoffrey, Prof. Dr., Habsburgerallee 11, 52064 Aachen (DE)
(74) Vertreter: Volmer, Georg

(56) Entgegenhaltungen:
- WO-A-99/45843
- US-A- 3 937 965
- US-A- 4 149 247
- US-A- 4 384 209
- US-A- 4 751 722
- GRANT JA, MORGAN MJ, DAVIS JR, WELLS P: "Reconstruction strategy suited to x-ray diffraction tomography" OPTICAL SOCIETY OF AMERICA, ISSN 0740-3232, Bd. 12, Nr. 2, - Februar 1995 (1995-02) Seiten 291-300, USA

## Beschreibung

Die Erfindung betrifft einen Computertomographen mit einer Abtasteinheit, die relativ zu einem Untersuchungsbereich um eine durch den Untersuchungsbereich verlaufende Rotationsachse drehbar ist und die eine Strahlenquelle zur Erzeugung eines Strahlenbündels, eine zwischen der Strahlenquelle und dem Untersuchungsbereich befindliche Blenden-Anordnung zur Erzeugung eines den Untersuchungsbereich durchsetzenden Strahlenfächers aus dem Strahlenbündel und eine zweidimensionale, eine Vielzahl von Detektorelementen umfassende Detektor-Anordnung umfasst, die mit einem Teil ihrer Messfläche Primärstrahlung aus dem Strahlenfächer und mit einem anderen Teil ihrer Messfläche in dem Untersuchungsbereich erzeugte Streustrahlung erfasst.

In einer Veröffentlichung von J.A. Grant et al "A RECONSTRUCTION STRATEGY SUITED TO X-RAY DIFFRACTION TOMOGRAPHY" Am.J.Optics A 12. 291-300, im folgenden auch kurz als D1 bezeichnet, ist ein Computertomographie-Verfahren bekannt, bei dem der Untersuchungsbereich entlang einer Vielzahl paralleler Strahlenpfade und aus einer Vielzahl von Richtungen mit einem bleistiftformigen Röntgenstrahl (pencil beam) durchstrahlt wird. Die Detektor-Anordnung jenseits des Untersuchungsbereichs erfasst dabei nicht nur den (geschwächten) Primärstrahl, sondern auch die elastisch gestreute Röntgenstrahlung. Elastisch bzw. kohärent gestreute Röntgenstrahlung entsteht bekanntlich, wenn die Röntgenquanten bei dem Streuprozess keine Energie verlieren, während die mit einem Energieverlust verbundene Form der Streuung als Compton-Streuung bezeichnet wird. Die elastische Streuung dominiert bei kleinen Streuwinkeln (z.B. < als 10°), während die Compton-Streuung bei den größeren Streuwinkeln überwiegt. Im Gegensatz zu der Compton-Streustrahlung erlaubt die elastisch gestreute Strahlung eine Charakterisierung der im Untersuchungsbereich befindlichen Materie.

Diejenigen Detektorelemente, die der Primärstrahlung nicht direkt ausgesetzt sind, werden von Streustrahlung getroffen, die entlang des jeweiligen Primärstrahls im gesamten Untersuchungsobjekt entsteht. Der Impulsübertrag (englisch: momentum transfer), der dem Produkt aus der Energie der gestreuten Quanten und dem Sinus des halben Streuwinkels proportional ist, lässt sich dabei mit Hilfe einer iterativen algebraischen Rekonstruktionstechnik (ART) rekonstruieren, das im einzelnen in D1 beschrieben ist. Als Ergebnis der Rekonstruktion wird für jedes Voxel im Untersuchungsbereich der von einem Primärstrahl durchsetzt wird, ein Impulsübertragsspektrum (das Impulsübertragsspektrum stellt die Intensität der Streustrahlung als Funktion des Impulsübertrages dar) erhalten, das für die Materie in dem betreffenden Voxek charakteristisch ist und somit Rückschlüsse über die stoffliche Zusammensetzung zulässt.

Ein in D1 bereits angesprochener Nachteil dieses Verfahrens besteht in der relativ langen Messzeit für die Akquisition sämtlicher für die Rekonstruktion erforderlicher Messdaten. In D1 ist angegeben, dass sich diese Messzeit durch einen Computertomographen z. B. mit einer Fächerstrahl-Geometrie verkürzen lässt, dass die Messdaten aber dann stärker durch unerwünschte Streustrahlung korrumpiert sind. Diesen Nachteil hat das aus der WO 99/45843 bekannte Verfahren. Jedes Detektorelement kann dabei Streustrahlung aus dem gesamten Streustrahlenfächer empfangen, also auch solche Streustrahlung, die unter einem relativ großen Streuwinkel gestreut wird - überwiegend also Compton-Streustrahlung, die keine Aussage über die stoffliche Zusammensetzung des Untersuchungsbereichs zulässt. Außerdem ist kein Rekonstruktionsverfahren bekannt, das unter solchen Voraussetzungen die Rekonstruktion der Streudichte im Untersuchungsbereich gestatten würde.

Aufgabe der vorliegenden Erfindung ist es daher, einen Computertomographen so auszugestalten, dass sich einerseits kurze Akquisitionszeiten und andererseits eine einwandfreie Rekonstruktion des Impulsübertragsspektrums im Untersuchungsbereich ergibt. Ausgehend von einem Computertomographen der eingangs genannten Art wird diese Aufgabe erfindungsgemäß dadurch gelöst, dass zwischen dem Untersuchungsbereich und der Detektor-Anordnung eine Kollimator-Anordnung mit einer Vielzahl von Lamellen angeordnet ist, die in Ebenen liegen, die den Strahlenfächer in eine Anzahl von Abschnitten unterteilen, so dass die in einer zur Rotationsachse parallelen Spalte befindlichen Detektorelemente von Primär- bzw. Streustrahlung aus dem gleichen Abschnitt getroffen werden. Die Kollimator-Anordnung hat bei der Erfindung eine Doppelfunktion:
a) Sie verhindert, dass unter einem größeren Streuwinkel gestreute Streustrahlung - überwiegend also Compton-Streustrahlung - auf die Detektorelemente trifft.
b) Sie unterteilt den Strahlenfächer in eine Anzahl von Abschnitten, die näherungsweise als "pencil beam" anzusehen sind, so dass die Impulsübertmgsspektren für die verschiedenen Voxel im Untersuchungsbereich nach dem aus D1 bekannten Verfahren rekonstruiert werden können.

Grundsätzlich gibt es verschiedene Möglichkeiten, wie man die parallel zur Rotationsachse bzw. senkrecht zum Strahlenfächer verlaufenden Lamellen der Kollimator-Anordnung anordnen kann. Anspruch 2 gibt die bevorzugte Anordnung an.

Die weitere Ausgestaltung gemäß Anspruch 3 macht es möglich, mit geringem zusätzlichen Aufwand einen Computertomographen einerseits zur Ermittlung der Impulsübertragsspektren der Streustrahlung und andererseits zur Ermittlung der Schwächung der Röntgenstrahlung -mit noch weiter verkürzter Akquisitionszeit (cone beam CT) - zu benutzen. Die weitere Ausgestaltung nach Anspruch 4 beschreibt den dafür nötigen Übergang von einem Strahlenfächer auf einen Strahlenkonus. Anspruch 5 erläutert die Verarbeitung der in den verschiedenen Betriebsmodi gewonnenen unterschiedlichen Messdaten.

Die Erfindung wird nachstehend anhand der Zeichnungen näher erläutert.
Es zeigen:
- Fig. 1: einen erfindungsgemäßen Computertomographen in schematischer Darstellung.
- Fig. 2: einen Querschnitt durch die Anordnung nach Fig. 1,
- Fig. 3: eine Draufsicht auf eine Spalte von detektorelementen und
- Fig. 4: ein Ablaufdiagramm zur Erläuterung der verschiedenen Betriebsmodi.

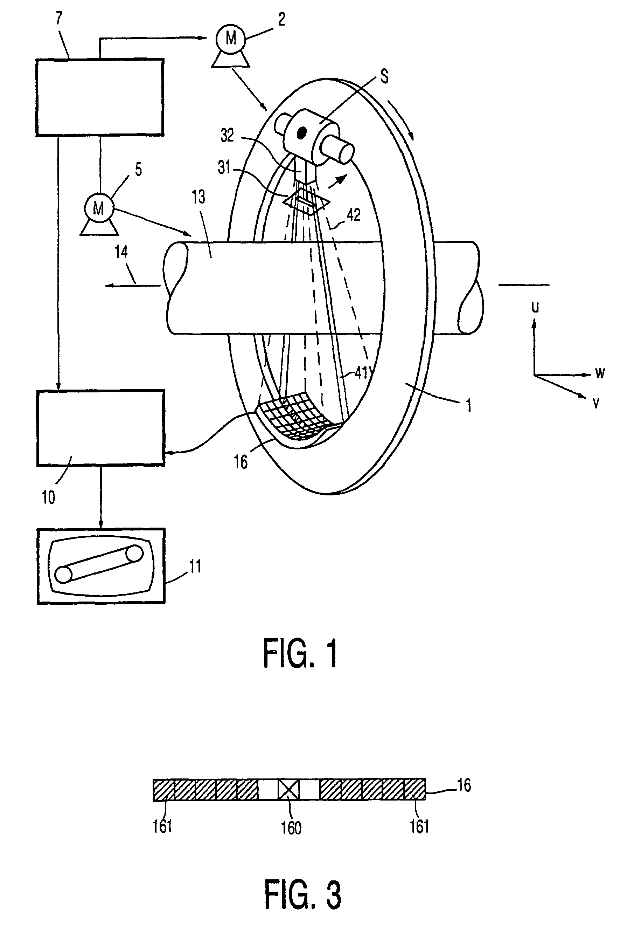

Der in Fig. 1 dargestellte Computertomograph umfasst eine Gantry 1, die um eine Rotationsachse 14 rotieren kann. Dazu wird die Gantry 1 von einem Motor 2 angetrieben. An der Gantry 1 ist eine Strahlenquelle S befestigt, beispielsweise ein Röntgenstrahler. Das zur Untersuchung benutzte Strahlenbündel wird von einer ersten Blendenanordnung 31 und/oder einer zweiten Blendenanordnung 32 bestimmt. Wenn die erste Blendenanordnung 31 wirksam ist, ergibt sich der mit ausgezogenen Linien dargestellte Strahlenfächer, der senkrecht zur Rotationsachse 14 verläuft und in deren Richtung geringe Abmessungen (z.B. 1mm) hat. Ist hingegen nur die zweite Blendenvorrichtung 32 im Strahlengang wirksam, dann ergibt sich der mit gestrichelten Linien angedeutete Strahlenkonus 42, der in einer zur Rotationsachse 14 senkrechten Ebene die gleiche Form hat, wie der Strahlenfächer 41, der aber in Richtung der Rotationsachse 14 wesentlich größere Abmessungen hat.

Das Strahlenbündel 41 bzw. 42 durchdringt einen zylinderförmigen Untersuchungsbereich 13, in dem sich z.B. ein Patient auf einem Patientenlagerungstisch (beides nicht näher dargestellt) oder aber auch ein technisches Objekt befinden kann. Nach dem Durchdringen des Untersuchungsbereichs 13 trifft das Strahlenbündel 41 bzw. 42 auf eine an der Gantry 1 befestigte zweidimensionale Detektor-Anordnung 16, die eine Vielzahl von matrixförmig angeordneten Detektorelementen umfasst. Die Detektorelemente sind in Zeilen und Spalten angeordnet. Die Detektorspalten verlaufen parallel zur Rotationsachse, die Detektorzeilen können sich in zur Rotationsachse senkrechten Ebenen befinden, beispielsweise auf einem Kreisbogen um die Strahlenquelle S. Die Detektorzeilen enthalten in der Regel wesentlich mehr Detektorelemente (z.B. 1000) als die Detektorspalten (z.B. 16).

Die Strahlenbündel 41 und 42, der Untersuchungsbereich 13 und die Detektor-Anordnung 16 sind aneinander angepasst. In einer zur Rotationsachse senkrechten Ebene 14 sind die Abmessungen dieses Strahlenfächers 41 bzw. des Strahlenkonus 42 so gewählt, dass der Untersuchungsbereich 13 vollständig durchstrahlt wird, und die Länge der Zeilen der Detektor-Anordnung ist gerade so bemessen, dass die Strahlenbündel 41 bzw. 42 vollständig erfasst werden können. Der Strahlenkonus 42 ist entsprechend der Länge der Detektorspalten gewählt, so dass der Strahlenkonus vollständig von der Detektor-Anordnung 16 erfasst werden kann. Wenn lediglich der Strahlenfächer 41 den Untersuchungsbereich durchstrahlt, trifft er auf den bzw. auf die mittlere(n) Detektorzeile(n).

Wenn es sich um ein technisches Objekt handelt und nicht um einen Patienten, kann das Objekt bei einer Untersuchung gedreht werden, während die Strahlenquelle S und die Detektor-Anordnung 15 still stehen. Das Objekt kann mittels eines Motors auch parallel zur Rotationsachse 14 verschoben werden. Wenn die Motoren 5 und 2 gleichzeitig laufen, ergibt sich eine helixförmige Abtastbewegung der Strahlenquelle S und der Detektor-Anordnung 16.

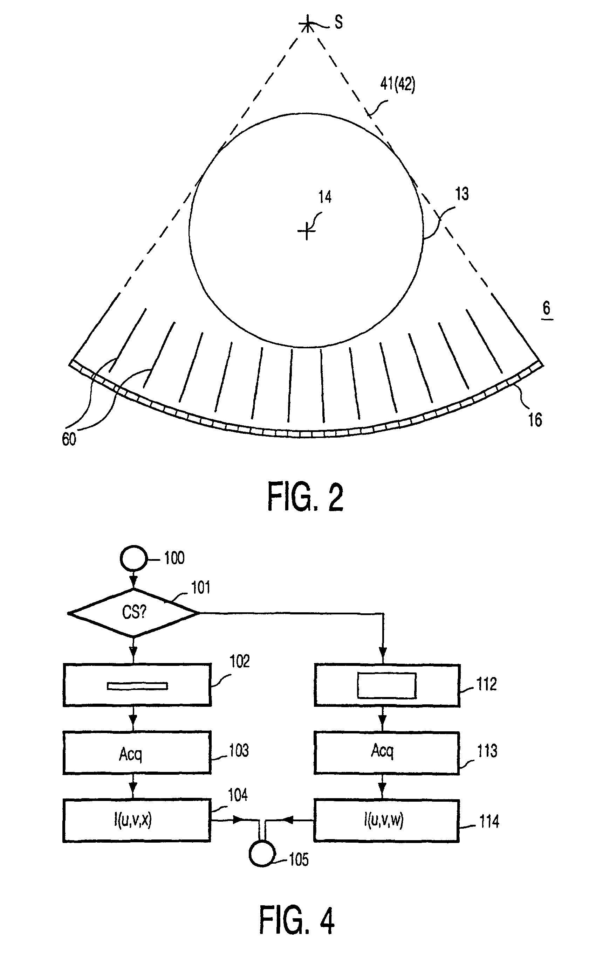

Wie aus Fig. 2 ersichtlich, befindet sich zwischen dem Untersuchungsbereich 13 und der Detektor-Anordnung 16 eine Kollimator-Anordnung 6, die eine Vielzahl ebener Lamellen 60 umfasst. Die Lamellen bestehen aus einem die Röntgenstrahlung stark absorbierenden Material und liegen in Ebenen, die parallel zur Rotationsachse 14 verlaufen und sich im Fokus der Strahlenquelle S schneiden. Ihr Abstand voneinander kann z.B. 1 cm betragen, und jede Lamelle kann in der Zeichenebene eine Abmessung von z.B. 20 cm haben. Durch die Kollimator-Anordnung 6 wird also der Strahlenfächer 41 in eine Anzahl von einander benachbarten Abschnitten unterteilt, so dass eine Spalte von Detektorelementen im wesentlichen nur von Primär- bzw. Streusuahlung aus einem Abschnitt getroffen werden kann.

Die von der Detektoreinheit 16 auf der rotierenden Gantry 1 akquirierten Messdaten werden einem Bildverarbeitungsrechner 10 zugeführt, der sich in der Regel an einem festen Punkt im Raum befindet und mit der Detektoreinheit über einen kontaktlos arbeitenden, nicht näher dargestellten Datenschleifring verbunden ist. Der Bildverarbeitungsrechner 10 kann verschiedene Bildverarbeitungsoperationen durchführen.

Im folgenden sollen die verschiedenen mit dem dargestellten Computertomographen möglichen Betriebsmodi anhand des in Fig. 4 dargestellten Ablaufdiagramms erläutert werden.

Nach der Initialisierung im Block 100 erfolgt im Schritt 101 eine Wahl des Betriebsmodus. Wenn der erste Betriebsmodus (in dem die Streustrahlung ausgewertet wird) gewählt wird, verzweigt der Ablauf zum Schritt 102, in dem einerseits die erste Blendenanordnung 31 und andererseits die Kollimator-Anordnung 6 in den Strahlengang gefahren werden. Zur Akquisition der Messwerte im Schritt 103 wird daher der Strahlenfächer 41 erzeugt. Während der Akquisition rotiert die Gantry, so dass die Detektorelemente die Primärstrahlung bzw. die Streustrahlung aus einer Vielzahl von Winkelpositionen erfassen. Das oder die Detektorelemente in der Mitte einer jeden Detektorspalte erfassen die Primärstrahlung, während die Streustrahlung (Sekundärstrahlung) von den in einer Spalte weiter außen liegenden Detektorelementen erfasst wird.

Dies ist in Fig. 3 schematisch angedeutet, die eine Draufsicht auf eine Spalte von Detektorelementen darstellt. Die Detektorelemente 161, die die Streustrahlung erfassen, sind einfach schraffiert dargestellt, während das Detektorelement 160 (bzw. die Detektorelemente) im Zentrum, das die Primärstrahlung erfasst durch ein Kreuz markiert ist. Beiderseits dieses zentralen Detektorelementes befinden sich auf Grund der endlichen Abmessungen des Fokus der Strahlenquelle Detektorelemente, die von Streustrahlung aber auch von einer (reduzierten) Primärstrahlung getroffen werden. Es ist zweckmäßig die Signale dieser in der Zeichnung nicht schraffiert oder gekreuzt dargestellten Detektorelemente bei der Auswertung nicht zu berücksichtigen.

Der Impulsübertrag X, dessen Spektrum als Funktion des Ortes u, v rekonstruiert werden soll, ist bekanntlich dem Produkt aus der Energie der gestreuten Röntgenquanten und den Sinus des halben Streuwinkels proportional (der Streuwinkel ist der Winkel, den die Bahn des gestreuten Röntgenquants mit der Bahn einschließt, die das Röntgenquant ohne den Streuprozess genommen hätte). Die weiter außen liegenden Detektorelemente erfassen die Streustrahlung im Mittel unter einem größeren Streuwinkel als die weiter innen liegenden.

Um den Impulsübertrag bestimmen zu können, muss einerseits der Streuwinkel und andererseits die Energie des gestreuten Röntgenquants bekannt sein. Der Streuwinkel ist gegeben durch die Lage des Detektorelements und die Lage des Punktes in dem Primärstrahlenfächer, in den der Streuprozess stattgefunden hat. Die Energie der gestreuten Röntgenquanten muss entweder gemessen werden, was voraussetzt, dass die Detektorelemente Energie aullösend messen können, oder es muss Röntgenstrahlung mit Quantenenergien aus einem möglichst kleinen Bereich (im Idealfall monochromatische Röntgenstrahlung) verwendet werden. Um die Energiedifferenz ΔE der Röntgenquanten möglichst klein in bezug auf ihre Energie E zu machen, gibt es verschiedene Möglichkeiten:
- Der Gebrauch geeigneter Filtermaterialien (z.B. Kupfer) im Primärstrahl. Dadurch wird die von einem Röntgenstrahler erzeugte weiche Röntgenstrahlung (das ist Röntgenstrahlung mit niedriger Quantenenergie) weitgehend unterdrückt.
- Zusätzlich kann man bei einer Röntgenröhre die Spannung im Hinblick auf die Filterwahl optimieren.
- Schließlich ist es möglich, die sogenannte "balanced filter" Technik anzuwenden. Dabei werden die Daten zweimal akquiriert, wobei sich im Strahlengang jeweils Filter mit leicht voneinander abweichender Ordnungszahl befinden, deren K-Kante zur Filterung herangezogen wird. Anschließend wird das Differenzsignal aus den beiden Messungen extrahiert.

Im abschließenden Schritt 104 wird das ortsabhängige Impulsübertragsspektrum rekonstruiert. Da die Lamellen den Strahlenfächer in eine Anzahl von Abschnitten zerlegen, von denen jeder zumindestens näherungsweise die Form eines "pencil-beam" hat, kann die Rekonstruktion analog zu dem in D1 beschriebenen Verfahren erfolgen. Damit ist das Verfahren beendet (Block 105).

Wenn im Schritt 101 hingegen der zweite Betriebsmodus gewählt wird, in dem lediglich die Schwächung der Primärstrahlung im Untersuchungsbereich rekonstruiert wird, verzweigt der Ablauf zum Block 112, in dem die erste Blendenanordnung 31 aus dem Strahlengang gefahren wird, so dass nur noch die zweite Blendenanordnung 32 wirksam ist, die einen Strahlenkonus 42 erzeugt. Außerdem wird im Block 112 der Kollimator 6 aus dem Bereich zwischen der Detektor-Anordnung 16 und dem Untersuchungsbereich 13 entfernt.

Bei der danach beginnenden Akquisition der Messdaten rotiert die Gantry um die Rotationsachse, wobei sämtliche Detektorelemente von Primärstrahlung getroffen werden können (Block 113). Im anschließenden Rekonstruktionsschritt 114 wird die Schwächung in einer Scheibe des Untersuchungsbereichs rekonstruiert. Ein geeignetes Rekonstruktionsverfahren ist in der deutschen Patentanmeldung 198451334 (PHD 98-123) beschrieben.

## Patentansprüche

1. Computertomograph mit einer Abtasteinheit, die relativ zu einem Untersuchungsbereich (13) um eine durch den Untersuchungsbereich verlaufende Rotationsachse (14) drehbar ist und die
- eine Strahlenquelle (S) zur Erzeugung eines Strahlenbündels,
- eine zwischen der Strahlenquelle und dem Untersuchungsbereich befindliche Blenden-Anordnung (31, 32) zur Erzeugung eines den Untersuchungsbereich durchsetzenden Strahlenfächers (41, 42) aus dem Strahlenbündel,
- eine zweidimensionale, eine Vielzahl von Detektorelementen umfassende Detektor-Anordnung (16), die mit einem Teil ihrer Messfläche Primärstrahlung aus dem Strahlenfächer und mit einem anderen Teil ihrer Messfläche in dem Untersuchungsbereich erzeugte Streustrahlung erfasst, und
- eine zwischen dem Untersuchungsbereich (13) und der Detektor-Anordnung (16) angeordnete Kollimator-Anordnung (6) mit einer Vielzahl von Lamellen, die in Ebenen liegen, die den Strahlenfächer in eine Anzahl von Abschnitten unterteilen, so dass die in einer zur Rotationsachse parallelen Spalte befindlichen Detektorelemente im wesentlichen nur von Primär- bzw. Streustrahlung aus ein und demselben Abschnitt getroffen werden,
umfasst und mit einem Büdverarbeitungsrechner (10) zur Bestimmung von Impulsübertragungsspektren für verschiedene Voxel aus dem Untersuchungsbereich (13).

2. Computertomograph nach Anspruch 1,
**dadurch gekennzeichnet,**
**dass** die Ebenen, in denen die Lamellen liegen, sich im Fokus der Strahlenquelle schneiden.

3. Computertomograph nach Anspruch 1,
**dadurch gekennzeichnet**,
**gekennzeichnet durch** einen ersten Betriebsmodus, in dem ein Teil der Detektorelernente die Streustrahlung erfasst, die in dem Strahlenfächer erzeugt wird, und **durch** einen zweiten Betriebsmodus, in dem die Detektorelemente die Primärstrahlung erfassen, die in einem Strahlenkonus erzeugt werden, dessen Abmessungen in Richtung der Rotationsachse größer sind als die des Sirahlenfachers.

4. Computertomograph nach Anspruch 3,
**dadurch gekennzeichnet,**
**dass** zwischen der Strahlenquelle und dem Untersuchungsbereich im ersten Betriebsmodus eine erste Blenden-Anordnung zur Erzeugung des Strahlenfächers und im zweiten Betriebsmodus eine zweite Blenden-Anordnung zur Erzeugung des Strahlenkonus im Strahlengang wirksam ist.

5. Computertomograph nach Anspruch 3,
**gekennzeichnet durch**
ein erstes Computerprogramm zur Berechnung der Streudichteverteilung in dem vom Strahlenfächer durchsetzten Teil des Untersuchungsbereichs aus den im ersten Betriebsmodus akquirierten Detektor-Signalen und **durch** ein zweites Computerprogramm zur Berechnung der Schwächung der Primärstrahlung in dem vom Strahlenkonus durchsetzten Teil des Untersuchungsbereichs aus den im zweiten Betriebsmodus akquirierten Detektor-Signalen.

## Revendications

1. Tomographe informatisé avec une unité de balayage qui peut tourner par rapport à une zone d'examen (13) autour d'un axe de rotation (14) traversant la zone d'examen et qui comprend
- une source de rayons (S) pour la production d'un faisceau de rayons,
- un dispositif de diaphragme (31, 32) se trouvant entre la source de rayons et la zone d'examen pour la production d'un éventail de rayons (41, 42) traversant la zone d'examen à partir du faisceau de rayons,
- un agencement de détecteurs (16) bidimensionnel, comprenant plusieurs éléments détecteurs qui saisit, avec une partie de sa surface de mesure, le rayonnement primaire de l'éventail de rayons et, avec une autre partie de sa surface de mesure, le rayonnement diffusé produit dans la zone d'examen et
- un dispositif de collimateur (6) disposé entre la zone d'examen (13) et l'agencement de détecteurs (16) avec plusieurs lamelles qui sont disposées dans des plans qui subdivisent l'éventail de rayons en plusieurs segments de telle sorte que les éléments détecteurs se trouvant dans une colonne parallèle à l'axe de rotation soient essentiellement touchés seulement par le rayonnement primaire ou le rayonnement diffusé d'un seul et même segment,
et avec un calculateur de traitement d'image (10) pour la détermination de spectres de transmission d'impulsion pour différents voxels à partir de la zone d'examen (13).

2. Tomographe informatisé selon la revendication 1,
**caractérisé en ce**
**que** les plans dans lesquels sont situés les lamelles se coupent dans le foyer de la source de rayons.

3. Tomographe informatisé selon la revendication 1,
**caractérisé par**
un premier mode de fonctionnement dans lequel une partie des éléments détecteurs saisit le rayonnement diffusé qui est produit dans l'éventail de rayons et par un deuxième mode de fonctionnement dans lequel les éléments détecteurs saisissent le rayonnement primaire qui est produit dans un premier cône de rayons dont les dimensions dans la direction de l'axe de rotation sont plus grandes que celles de l'éventail de rayons.

4. Tomographe informatisé selon la revendication 3,
**caractérisé en ce**
**qu'**un premier dispositif de diaphragme est actif entre la source de rayons et la zone d'examen dans le premier mode de fonctionnement pour la production de l'éventail de rayons et un deuxième dispositif de diaphragme est actif dans le deuxième mode de fonctionnement pour la production du cône de rayons dans le trajet des rayons.

5. Tomographe informatisé selon la revendication 3,
**caractérisé par**
un premier programme informatique pour le calcul de la distribution de la densité diffusée dans la partie de la zone d'examen traversée par l'éventail de rayons à partir des signaux de détecteur acquis dans le premier mode de fonctionnement et par un deuxième programme informatique pour le calcul de l'atténuation du rayonnement primaire dans la partie de la zone d'examen traversée par le cône de rayons à partir des signaux de détecteur acquis dans le deuxième mode de fonctionnement.

## Claims

1. A computed tomography apparatus which is rotatable relative to an examination zone (13), about an axis of rotation (14) and which comprises
- a radiation source (S) for generating a radiation beam,
- a diaphragm arrangement (31, 32) which is arranged between the radiation source and the examination zone in order to form a fan beam (41, 42) traversing the examination zone from the radiation beam,
- a two-dimensional detector arrangement (16) which includes a plurality of detector elements and a part of the measuring surface of which detects primary radiation from the fan beam whereas another part of its measuring surface detects scattered radiation produced in the examination zone, and
- between the examination zone (13) and the detector arrangement (16) a collimator arrangement (6) which includes a plurality of lamellas which are situated in planes that subdivide the fan beam into a number of segments so that the detector elements that are situated in a column extending parallel to the axis of rotation essentially are struck only by primary radiation or scattered radiation from one and the same segment,
and comprises an image processing computer (10) for determining momentum transfer spectra for various voxels in the examination zone (13).

2. A computed tomography apparatus as claimed in claim 1,
**characterized in that** the planes in which the lamellas are situated intersect at the focus of the radiation source.

3. A computed tomography apparatus as claimed in claim 1,
**characterized in that** it has a first mode of operation in which a part of the detector elements detects the scattered radiation that is generated in the fan beam, and a second mode of operation in which the detector elements detect the primary radiation that is generated in a cone beam whose dimensions in the direction of the axis of rotation are larger than those of the fan beam.

4. A computed tomography apparatus as claimed in claim 3,
**characterized in that** in the first mode of operation a first diaphragm arrangement is arranged between the radiation source and the examination zone in order to generate the fan beam whereas in the second mode of operation a second diaphragm arrangement is active in the beam path in order to generate the cone beam.

5. A computed tomography apparatus as claimed in claim 3,
**characterized in that** it involves a first computer program for calculating the scatter density distribution in the part of the examination zone traversed by the fan beam from the detector signals acquired in the first mode of operation, and also a second computer program for calculating the attenuation of the primary radiation in the part of the examination zone traversed by the cone beam from the detector signals acquired in the second mode of operation.
